# EUROPEAN PATENT APPLICATION

(11) **EP 4 818 186 A1**
(43) Date of publication of application: **07.10.2026**
(21) Application number: 25168299.3
(22) Date of filing: 03.04.2025
(51) Int. Cl.: C07K 1/107, C07K 1/00, C07K 14/78, C07K 2/00, C07K 4/12

(54) **A METHOD FOR PREPARING A TELECHELIC POLYMER FROM A PEPTIDE**

(71) Applicant: Matrihealth GmbH, 06120 Halle (Saale) (DE)
(72) Inventor: LEHMANN, Florian, 06120 Halle (Saale) (DE); HEDTKE, Tobias, 06120 Halle (Saale) (DE)
(74) Representative: Maiwald GmbH

(57) **Abstract**

A method for preparing a telechelic polymer derived from a peptide, wherein the telechelic polymer is suitable for end-crosslinking, the method comprising the steps of:
- Providing in a *peptide provision step* a peptide, preferably a polypeptide, comprising an N-terminus comprising an amino group and a carboxy-terminus comprising a carboxylic acid group;
- Chemically activating in a *carboxy-terminus activation step* the carboxylic acid group of the carboxy-terminus of said peptide yielding a peptide, preferably a polypeptide, comprising an activated carboxy-terminus;
- Reacting in an *activated carboxy-terminus nucleophile introduction step* said peptide, preferably polypeptide, comprising an activated carboxy-terminus with a compound comprising a nucleophile yielding a peptide, preferably a polypeptide, comprising a modified carboxy-terminus, wherein the modified carboxy-terminus comprises the nucleophile; and
- Reacting in an *N-terminus cross-linkable modification step* said N-terminus with an N-terminus specific cross-linkable compound, thereby yielding a cross-linkable N-terminus;
- Reacting in a *modified carboxy-terminus cross-linkable modification step* said modified carboxy-terminus with a modified carboxy-terminus-specific cross-linkable compound, thereby yielding a cross-linkable modified carboxy-terminus;
wherein the N-terminus cross-linkable modification step can be carried out prior to the modified carboxy-terminus cross-linkable modification step, the N-terminus cross-linkable modification step can be carried out subsequent to the modified carboxy-terminus cross-linkable modification step, or the N-terminus cross-linkable modification step can be carried out at the same time as the modified carboxy-terminus cross-linkable modification step, thereby yielding a peptide, preferably polypeptide, comprising a cross-linkable N-terminus and a cross-linkable modified carboxy-terminus.

## Description

### Technical Field of the Invention

The invention relates to a method for the preparation of a polymer suitable for radical cross-linking, such as stereo lithography, especially to a method for the preparation of a polymer suitable for mimicking the function and mechanical properties of the extracellular matrix of a tissue after cross-linking. Moreover, the invention relates to a composition comprising a polymer suitable for radical cross-linking, such as stereo lithography, especially to a composition comprising a polymer suitable for mimicking the function and mechanical properties of the extracellular matrix of a tissue after cross-linking. The present invention further concerns a method for preparing an article using said composition by radical cross-linking process steps, such as stereolithography, digital light processing, and continuous liquid interface production.

### Background of the Invention

Polypeptides are widely utilized in biomedical applications due to their biocompatibility, biodegradability, and ability to be functionalized for specific biological interactions. In tissue engineering and biomaterial development, the modification of polypeptides is essential to tailor their mechanical properties, bioactivity, and stability under physiological conditions. These modifications enable the creation of scaffolds and matrices that mimic the extracellular environment, promoting cell adhesion, proliferation, and tissue regeneration.

Common materials used in these applications include collagen, elastin, fibrin, and synthetic peptides, each offering distinct advantages and presenting unique challenges. Collagen provides excellent biocompatibility and structural support but may suffer from batch variability and limited mechanical strength. Fibrin is advantageous for promoting cell adhesion and rapid degradation, but its mechanical integrity is often insufficient for long-term applications. Synthetic peptides offer precise control over composition and functionality but can lack the natural bioactivity and complexity of native proteins.

Elastin plays a crucial role in tissue engineering due to its exceptional elasticity and resilience, which are essential for replicating the mechanical properties of dynamic tissues such as skin, blood vessels, and lungs. Elastin-based biomaterials can provide durable, stretchable matrices that withstand cyclic mechanical stress while maintaining biocompatibility. Elastin is an insoluble protein formed in vivo by the polymerization of tropoelastin, a soluble polypeptide precursor, through the crosslinking of its lysine residues. This process results in a robust, elastic structure capable of enduring repeated mechanical deformation without compromising its integrity. Elastin allows for highly tunable hydrogel formulations, particularly enhancing the elastic modulus beyond what is achievable with other natural polymers like gelatin or collagen.

A significant area of interest involves replicating these advantageous properties of elastin in tissue engineering applications. One strategy developed in the prior art is to use recombinant tropoelastin or elastin-like peptides (ELPs) produced by genetically modified organisms. These peptides are typically crosslinked in situ shortly after 3D bioprinting. For example, Advanced Materials 32(45) (2020) 1-10 describes a recombinant tropoelastin functionalized with methacrylic groups combined with gelatin-methacrylate in photocrosslinkable bioinks for printing vascularized tissues with high cell viability. However, recombinant tropoelastin and ELPs are expensive and obtainable only in low yields, limiting their practical application on a larger scale.

To overcome these limitations, efforts have been made to utilize natural elastin, which is abundantly available as a byproduct of the meat production sector. For instance, ACS Omega 6(33) (2021) 21368-21383 reports the use of elastin extracted from bovine neck ligament as a component of biomaterial inks. In this study, elastin was added in ground form at low concentrations (e.g., 1 wt% elastin with 10 wt% GelMA or 0.5 wt% collagen) due to its poor solubility in water. This approach is limited by the inability to incorporate higher elastin content, restricting the material's mechanical and biological performance.

EP 4 338 763 A1 proposes the use of water-soluble elastin, preferably produced by the hydrolyzation of natural elastin, as a solution to these issues. Elastin is primarily composed of hydrophobic amino acids and features repetitive sequence motifs such as VPGVG, GVPGV, PGVGV, GVPGVG, PGVGVA, GVPG, GXPG, VPGXG, GXVPG, GXXPG, GXVPGX, where X represents any canonical amino acid except proline. Approximately 80% of elastin consists of glycine, alanine, proline, and valine, with lysine accounting for about 5% of the total amino acid composition. Lysine residues play a crucial role in crosslinking and stabilizing biomaterials due to the nucleophilic nature of their primary amines.

In naturally derived elastin, the majority (~90%) of lysine residues are already involved in crosslinking, rendering them inaccessible for further modification. Additionally, each tropoelastin entity contains a free N-terminal amine, which also serves as a potential site for crosslinking.

Despite these advantages, hydrolyzation also randomly cleaves the elastin main chain at peptide bonds, producing smaller fragments. Given the low lysine content in elastin, most of these fragments lack lysine residues, limiting their crosslinking potential to the lone N-terminus.

### Summary of the Invention

Consequently, the water-soluble elastin suggested in EP 4 338 763 A1 requires co-use with other cross-linkable biopolymers, such as methacrylated gelatin (GeIMA), to achieve effective crosslinking. However, these alternative biopolymers cannot replicate the unique mechanical and biological properties of elastin.

Therefore, improved methods for the modification of elastin and other polypeptides are required to enhance their mechanical properties, bioactivity, and applicability in advanced tissue engineering and biomaterial design. The present invention addresses these challenges by providing novel approaches to polypeptide modification, facilitating the development of enhanced biomaterials for medical and research applications.

In particular, a method is needed, which is able to provide at least two cross-linking sites in a polypeptide.

It has now been surprisingly found that above-mentioned objects can be achieved by a method for preparing a telechelic polymer derived from a peptide, wherein the telechelic polymer is suitable for end-crosslinking, the method comprising the steps of:
- Providing in a *peptide provision step* a peptide, preferably a polypeptide, comprising an N-terminus comprising an amino group and a carboxy-terminus comprising a carboxylic acid group;
- Chemically activating in a *carboxy-terminus activation step* the carboxylic acid group of the carboxy-terminus of said peptide yielding a peptide, preferably a polypeptide, comprising an activated carboxy-terminus;
- Reacting in an *activated carboxy-terminus nucleophile introduction step* said peptide, preferably polypeptide, comprising an activated carboxy-terminus with a compound comprising a nucleophile yielding a peptide, preferably a polypeptide, comprising a modified carboxy-terminus, wherein the modified carboxy-terminus comprises the nucleophile; and
- Reacting in an *N-terminus cross-linkable modification step* said N-terminus with an N-terminus specific cross-linkable compound, thereby yielding a cross-linkable N-terminus;
- Reacting in a *modified carboxy-terminus cross-linkable modification step* said modified carboxy-terminus with a modified carboxy-terminus-specific cross-linkable compound, thereby yielding a cross-linkable modified carboxy-terminus;

wherein the N-terminus cross-linkable modification step can be carried out prior to the modified carboxy-terminus cross-linkable modification step, the N-terminus cross-linkable modification step can be carried out subsequent to the modified carboxy-terminus cross-linkable modification step, or the N-terminus cross-linkable modification step can be carried out at the same time as the modified carboxy-terminus cross-linkable modification step,
thereby yielding a peptide, preferably polypeptide, comprising a cross-linkable N-terminus and a cross-linkable modified carboxy-terminus.

Furthermore, it has now been surprisingly found that above-mentioned object is also achieved by a composition comprising a telechelic polymer derived from a peptide, wherein the telechelic polymer is suitable for end-crosslinking, and an activatable cross-linking agent.

Finally, it has been surprisingly found that above-mentioned objects can be achieved by a method for preparing an article from a composition according to the present invention, wherein the method is selected from the list consisting of stereolithography, digital light processing, xolography, and continuous liquid interface production.

The advantage of the present invention is that polypeptides, which have no cross-linkable group in the side chains of the amino acid units in the main chain, can be modified to be at least head-tail cross-linkable. This is achieved by an artificial increase of the cross-linkable amino groups of the polypeptide by modification of the carboxy-terminus. If the polypeptide originally has only one cross-linkable group in form of the free amino group at the N-terminus, this results in doubling the numbers of cross-linkable groups in the polypeptide and ensures cross-linkability. If other cross-linkable groups are present i.e. in the side chains of amino acid units in the main chain, the method of the present invention still works and also adds one more cross-linkable group to the number of cross-linkable groups present in the original polypeptide.

Thus, the present invention has the advantage that polypeptides, which have not more than one cross-linkable group can be transformed to cross-linkable peptide derivatives, which allows for self-cross-linking of said derivative instead of using a second, cross-linkable polymer. Thereby, specific properties of the derivative can be solely used in a cross-linked material, such as a hydrogel.

### Short Description of the Drawings

- Figure 1: is a schematic representation of the respective structure of N-(2-aminoethyl)elastinpeptidylamide (EL-EDA) as prepared in IE1 and IE2. The elastin peptide chain is depictured as a black, bold, and solid line.
- Figure 2: is a schematic representation of the respective structure of N-(6-aminohexyl)elastinpeptidylamide (EL-HMDA) as prepared in IE3. The elastin peptide chain is depictured as a black, bold, and solid line.
- Figure 3: is a schematic representation of the respective structure of N-(8-amino-3,6-dioxaoctyl)elastinpeptidylamide (EL-2ODA) as prepared in IE4. The elastin peptide chain is depictured as a black, bold, and solid line.
- Figure 4: is a schematic representation of the respective structure of N-(13-amino-4,7,10-trioxatridecanoyl)elastin-peptidylamide (EL-3ODA) as prepared in IE5. The elastin peptide chain is depictured as a black, bold, and solid line.
- Figure 5: is a schematic representation of the respective structures of terminally functionalized peptides comprising elastin methacrylamide (EL-EDA-MA) as prepared in IE6. The elastin peptide chain is depictured as a black, bold, and solid line.
- Figure 6: is a schematic representation of the respective structures of terminally functionalized peptides comprising elastin methacrylamide (EL-2ODA-MA) as prepared in IE7. The elastin peptide chain is depictured as a black, bold, and solid line.
- Figure 7: is a schematic representation of the respective structures of terminally functionalized peptides comprising elastin methacrylamide (EL-3ODA-MA) as prepared in IE8. The elastin peptide chain is depictured as a black, bold, and solid line.
- Figure 8: is a schematic representation of the respective structures of terminally functionalized peptides comprising elastin itaconylamide (EL-EDA-IA) prepared from EL-EDA as prepared in IE9. The elastin peptide chain is depictured as a black, bold, and solid line.

### Definitions

The term *"telechelic polymer"* as used herein denotes a polymer with reactive functional groups at both of its main chain ends. These terminal groups allow the polymer to participate in further chemical reactions, such as crosslinking or grafting onto other molecules, such as used for example in bioconjugation and respective applications thereof. Telechelic polymers are widely used in the synthesis of block copolymers, hydrogels, and other advanced materials due to their ability to form well-defined network structures. The nature of the functional groups can vary, including amines, carboxyl, hydroxyl, or other reactive moieties. This versatility makes telechelic polymers valuable in applications ranging from biomedical engineering to adhesives and coatings.

The term *"bioconjugation"* as used herein denotes the process of chemically linking two biomolecules or a biomolecule with another functional molecule, such as a synthetic polymer, nanoparticle, or drug to enhance the functionality, stability, or targeting ability of biomolecules, particularly in biomedical applications. Examples for bioconjugation include amide bond formation, thiol-maleimide conjugation, click chemistry (e.g., azide-alkyne cycloaddition), and bioorthogonal reactions.

The term *"main chain"* of a polymer as used herein refers to the continuous, linear sequence of covalently bonded atoms that form the primary structural framework of the polymer. It consists of repeating units derived from monomers and defines the overall length and architecture of the polymer. The main chain can be composed of carbon atoms (in organic polymers) or other elements such as silicon (in silicones) or nitrogen (in polyamides). Side groups or side chains may branch off from the main chain, influencing the polymer's physical and chemical properties. Usually, the main chain denotes the longest chain provided in the polymer. However, in polymers, which are defined by starting and end groups, such as a polypeptide, the main chain is also defined by the chain connecting these groups. The term *"main chain"* is also often referred to as *"backbone".*

The term *"peptide"* as used herein denotes a short chain of amino acids linked together by peptide bonds, which are covalent bonds formed between the carboxyl group of one amino acid and the amino group of another. Peptides typically consist of 2 to 50 amino acids, with chains longer than 50 amino acids generally classified as proteins or polypeptides.

The term *"N-terminus"* as used herein (typically also known as amino-terminus) of a polypeptide denotes the end of the chain that has a free amino group (-NH₂). It is the first amino acid in the sequence, as polypeptides are synthesized from the N-terminus to the C-terminus during translation.

The term *"carboxy-terminus"* as used herein (typically also known as C-terminus) of a polypeptide denotes the end of the chain that has a free carboxyl group (-COOH). It is the last amino acid added during polypeptide synthesis.

The term *"cross-linking"* as used herein in the context of polypeptides refers to the formation of covalent or non-covalent bonds between different polypeptide or polypeptide derivative chains or within the same chain, creating a three-dimensional network. This process stabilizes the structure, enhances mechanical strength, and improves resistance to degradation. In polypeptides, cross-linking typically occurs through reactive side-chain groups, such as the ε-amino group of lysine, the thiol group of cysteine, or through chemical modifications like photo-crosslinking or enzymatic reactions. Cross-linking is crucial in biomaterials, particularly for hydrogels, scaffolds, and drug delivery systems, as it tailors the mechanical and biological properties for tissue engineering and biomedical applications.

The term *"elastin"* as used herein denotes a biopolymer present in the extracellular matrix of vertebrates providing elasticity and resilience to tissues and organs. Elastin has outstanding characteristics such as a very low elastic modulus, a very high biological half-life and a high resistance to proteolytic enzymes. Elastin is isolated from vertebrate tissues (e. g. aorta, ligaments) and is insoluble in all organic and inorganic solvents. Elastin can be obtained in large amounts as a by-product of meat production. However, this elastin is insoluble in water and thus needs to be modified to be soluble.

The term *"native elastin"* as used herein denotes elastin as extracted from the extracellular matrix without any further modification.

The term *"gelatin"* as used herein denotes the irreversibly hydrolyzed form of collagen, wherein the hydrolysis reduces protein fibrils into smaller peptides.

The term *"collagen"* as used herein denotes the main structural protein in the extracellular matrix found tissues of mammals. As such, it is the most abundant protein in mammals, making up from 25% to 35% of the whole-body protein content. Collagen consists of amino acids bound together to form a triple helix of elongated fibril known as a collagen helix.

The term *"biocompatible"* as used herein denotes the ability of a material to perform with an appropriate host response in a specific application.

### Detailed Description of the Invention

The present invention concerns a method for preparing a telechelic polymer derived from a peptide, a composition comprising said telechelic polymer, and a method for preparing an article of said composition. These embodiments are described in more detail in the following.

### Method for preparing a telechelic polymer

As outlined above, the most generic embodiment of the method for preparing a telechelic polymer is a method for preparing a telechelic polymer derived from a peptide, wherein the telechelic polymer is suitable for end-crosslinking, the method comprising the steps of:
- Providing in a *peptide provision step* a peptide, preferably a polypeptide, comprising an N-terminus comprising an amino group and a carboxy-terminus comprising a carboxylic acid group;
- Chemically activating in a *carboxy-terminus activation step* the carboxylic acid group of the carboxy-terminus of said peptide yielding a peptide, preferably a polypeptide, comprising an activated carboxy-terminus;
- Reacting in an *activated carboxy-terminus nucleophile introduction step* said peptide, preferably polypeptide, comprising an activated carboxy-terminus with a compound comprising a nucleophile yielding a peptide, preferably a polypeptide, comprising a modified carboxy-terminus, wherein the modified carboxy-terminus comprises the nucleophile; and
- Reacting in an *N-terminus cross-linkable modification step* said N-terminus with an N-terminus specific cross-linkable compound, thereby yielding a cross-linkable N-terminus;
- Reacting in a *modified carboxy-terminus cross-linkable modification step* said modified carboxy-terminus with a modified carboxy-terminus-specific cross-linkable compound, thereby yielding a cross-linkable modified carboxy-terminus;

wherein the N-terminus cross-linkable modification step can be carried out prior to the modified carboxy-terminus cross-linkable modification step, the N-terminus cross-linkable modification step can be carried out subsequent to the modified carboxy-terminus cross-linkable modification step, or the N-terminus cross-linkable modification step can be carried out at the same time as the modified carboxy-terminus cross-linkable modification step,
thereby yielding a peptide, preferably polypeptide, comprising a cross-linkable N-terminus and a cross-linkable modified carboxy-terminus.

### a) The peptide provision step

Preferably, in the method for preparing a telechelic polymer according to the present invention, the *peptide provision step* comprises the step of unspecific hydrolysis of a natural occurring polypeptide, preferably unspecific hydrolysis of macromolecular elastin, most preferably unspecific hydrolysis of macromolecular elastin isolated from mammalian tissue.

Hence, preferably, the peptide, preferably polypeptide, provided in the peptide provision step of the method for preparing a telechelic polymer according to the present invention is a hydrolyzed elastin. In the step of unspecific hydrolysis, native elastin is hydrolyzed using chemical and/or enzymatic treatment thereby producing fragments of the elastin protein having different sizes. Hence, preferably, the *peptide provision step* of the method for preparing a telechelic polymer according to the present invention comprises, preferably consists of, hydrolyzing a native elastin by chemical, preferably acidic or basic, i.e. alkaline, or enzymatic hydrolysis.

The degree of hydrolyzation can be varied by the incubation time or the type and efficiency of the hydrolyzation agent. According to the hydrolyzation method, the distribution of the molecular weight of the resulting hydrolyzed elastin includes fragments with a molecular weight from as low as 1 kDa up to over 700 kDa. Besides controlling hydrolysis, the molecular weight distribution of hydrolyzed elastin can be defined by fractionation (e.g., membrane filtration with different molecular weight cut-off values) or by using more than one type of hydrolyzed elastin.

Particularly, partial acidic hydrolysis of native elastin leads to high molecular weight fragments, wherein the product of the produced hydrolyzed elastin is usually called α-elastin. α-elastin retains most of the physicochemical properties and the amino acid composition of native elastin. On the other hand, a fractionation process of α-elastin leads to a fraction with low molecular weight fragments, to which it is referred to as β-elastin. Alternatively, soluble elastin with low mean molecular weight can be obtained by basic hydrolysis, the product of which is referred to as κ-elastin.

Hence, more preferably, the hydrolyzed elastin provided in the *peptide provision step* of the method for preparing a telechelic polymer according to the present invention is selected from the list consisting of α-elastin, β-elastin, κ-elastin, or mixtures thereof. Due to the closest resembling of the properties of elastin, the hydrolyzed elastin most preferably is α-elastin.

Preferably, the native elastin used in the process of the present invention is extracted from animal tissues, more preferably vertebrate tissues, and most preferably mammalian tissues. Preferable mammalian tissues are selected from the list consisting of bovine, porcine, equine, murine, sheep, chicken, rat, avian, fish, and rabbit tissues as well as marine vertebrate tissues, more preferably tissue derived from porcine, bovine, avian, and fish. In special cases, donor tissues from human might also be considered.

### b) The carboxy-terminus activation step

Generally, in the *carboxy-terminus activation step* the carboxylic acid functional groups comprised in the carboxy-terminus of peptide, preferably polypeptide, is made susceptible to chemical modifications.

Preferably, in the *carboxy-terminus activation step,* a transient intermediate is formed by a carbodiimide in form of an O-acylisourea intermediate. The O-acylisourea residue is suitable for acting as a leaving group in the subsequent *activated carboxy-terminus nucleophile introduction step.*

Carbodiimides suitable for being used in the preferred embodiment of the *carboxy-terminus activation step* include both acyl and alkyl carbodiimides. Preferably, the carbodiimide is a carbodiimide according to formula (I):

R-N=C=N-R' (I)

wherein R and R' can be alkyl, aryl, or heteroaryl groups.

Preferred carbodiimides include those with electron-donating groups (e.g., dimethylamino groups) in the R' position, enhancing solubility and reactivity, especially in aqueous environments. Substituents on the carbodiimide's R or R' groups can further influence reactivity, with alkyl substitutions like methyl or ethyl being particularly beneficial for controlled activation.

For enhanced specificity and reduced side reactions, carbodiimides with bulky groups, preferably dicyclohexylcarbodiimide (DCC), are preferred in cases requiring minimal unwanted cross-linking.

More preferably, 1-ethyl-3-(3-dimethylaminopropyl)carbodiimide (EDC) is used, which offers a balance of reactivity, solubility, and compatibility with a wide array of peptide residues such as glutamic acid, aspartic acid, and serine. Thus, EDC is the most preferred carbodiimide for modifying C-terminal residues in peptide-based applications, due to its enhanced efficiency and mild reaction conditions.

Other methods for activating carboxylic acids present at the carboxy-terminus of peptides are known in the art. These methods can also be used in the *carboxy-terminus activation step* in the method for preparing a telechelic polymer according to the present invention. However, the route via carbodiimides is preferred. One of the other methods is, e.g., the activation of carboxylic acid functional groups by thionyl chloride.

Optionally, the *carboxy-terminus activation step* comprises the additional formation of stable intermediates of carboxy-terminally activated peptides from the transient intermediate, preferably in the form of activated esters or thioesters. Generally, thioesters are more potent leaving groups for the subsequent *activated carboxy-terminus nucleophile introduction step.* Nevertheless, sterically hindered esters can also work as such leaving groups.

The most common stable forms of the stable intermediate derived from the transient intermediate are N-hydroxysuccinimide (NHS) esters or sulfo-N-hydroxysuccinimide (sulfo-NHS) esters. These stable intermediates are synthesized by reacting the carbodiimide-activated carboxylic functional groups with an alcohol or a thiol, preferably with NHS or sulfo-NHS.

It should be noted that for a one-pot reaction, the reaction with carbodiimide is sufficient. However, in case a two-pot reaction should be carried out and/or the formed peptide, preferably a polypeptide, comprising an activated carboxy-terminus should be stored or isolated for other reasons, the formation of an ester or thioester can be optionally applied.

Hence, preferably, the *carboxy-terminus activation step* comprises a step selected from the list consisting of formation of an activated ester of the carboxy-terminus, formation of an acyl halide of the carboxy-terminus, activation of the carboxy-terminus by carbodiimides, formation of a mixed anhydride of the carboxy-terminus, and formation of a thioester of the carboxy-terminus. Most preferably, *carboxy-terminus activation step* comprises the step of activation of the carboxy-terminus by carbodiimides, activated ester formation, or activated thioester formation.

### c) The activated carboxy-terminus nucleophile introduction step

In the *activated carboxy-terminus nucleophile introduction step* the reactive chemical groups introduced to the carboxy terminus of carboxy-terminally activated peptides are nucleophiles.

The term *"nucleophile"* as used herein denotes a molecule or ion that donates a pair of electrons to form a new covalent bond during a chemical reaction. Nucleophiles are electron-rich species and are attracted to electron-deficient centers, such as positively charged or partially positive atoms.

In the activation of a carboxyl group, the compound comprising a nucleophile attacks the electrophilic carbon of the carbonyl group, initiating a nucleophilic substitution or addition-elimination reaction.

Preferred compounds comprising a nucleophile are selected from the list consisting of an amine (forming an amide), an alcohol (forming an ester), and a thiol (forming a thioesters).

More preferably, in the *activated carboxy-terminus nucleophile introduction step* the compound comprising a nucleophile is a primary amine. Even more preferably, the compound comprising a nucleophile is a diamine. This has the advantage that the reactivities of the modified carboxy-terminus and the N-termini of the peptide are unified. Most preferably, the compound comprising a nucleophile is selected from the list consisting of ethylene diamine, hexamethylene diamine, and 2,2'-(ethylenedioxy)bis(ethylamine). In such embodiment, the nucleophile is a primary amino group.

Consequently, preferably, in the *activated carboxy-terminus nucleophile introduction step* the nucleophile is introduced via amide coupling to the activated carboxy-terminus.

As set out above for the *carboxy-terminus activation step,* peptides having an activated carboxy-terminus can be formed as either stable or transient intermediates. Hence, the *carboxy-terminus activation step* and the *activated carboxy-terminus nucleophile introduction step* can be performed separately if stable intermediates are formed, or together in a *"one-pot reaction"* if transient intermediates are formed.

Most preferably, the *carboxy-terminus activation step* and the *activated carboxy-terminus nucleophile introduction step* are carried out in a *"one-pot reaction"* with the formation of transient intermediates.

### d) The modified carboxy-terminus cross-linkable modification step

In the *modified carboxy-terminus cross-linkable modification step,* the reactive chemical groups introduced to the modified carboxy-terminus are chemically functionalized by a modified carboxy-terminus-specific cross-linkable compound. Preferably, the modified carboxy-terminus-specific cross-linkable compound contains electrophilic chemical entities to react with the nucleophile introduced in the *activated carboxy-terminus nucleophile introduction step* to generate peptides with a cross-linkable carboxy-terminus. Thus, the modified carboxy-terminus-specific cross-linkable compound is suitable for specifically reacting with the modified carboxy-terminus.

In a preferred embodiment of the method for preparing a telechelic polymer of the present invention, said nucleophile is a primary amino group. Thus, in this preferred embodiment the modified carboxy-terminus-specific cross-linkable compound is a chemical entity, which is highly reactive against primary amines. Hence, preferably, in case the nucleophile is a primary amino group, the modified carboxy-terminus-specific cross-linkable compound is selected from the list consisting of aldehydes, epoxides (oxiranes), isocyanates, isothiocyanates, sulfonyl chlorides, carbodiimides, acyl azides, anhydrides, fluorobenzenes, carbonates, NHS esters, imidoesters, and fluorophenyl esters.

In one particularly preferred embodiment, carboxylic acid anhydrides are used as modified carboxy-terminus-specific cross-linkable compounds. The reaction of the carboxylic acid anhydride and the activated carboxy-terminus having a primary amino group as nucleophile forms an amide bond between the primary amino group and one carboxylic acid moiety from the carboxylic acid anhydride. Thereby, the carboxylic acid anhydride can be selected from cyclic and aliphatic carboxylic acid anhydrides. Cyclic anhydrides originating from a dicarboxylic acid do not generate free carboxylic acid byproducts during this reaction, whereas aliphatic carboxylic acid anhydrides release carboxylic acids as byproducts. Hence, cyclic anhydrides are preferred carboxylic acid anhydride. However, as the use of anhydrides will always result in the formation of carboxylic groups, which affect the pH value, in the modified carboxy-terminus cross-linkable modification step using anhydrides as modified carboxy-terminus-specific cross-linkable compounds preferably a pH adjustment step is carried out to maintain the deprotonated state of primary amino groups in the peptide.

In another particularly preferred embodiment, a carboxylic acid in combination with a carbodiimide, preferably EDC, is used as modified carboxy-terminus-specific cross-linkable compounds. This reaction forms an amide bond between the primary amino groups and a carboxylic acid moiety.

Hence, more preferably, in case the nucleophile is a primary amino group, the modified carboxy-terminus-specific cross-linkable compound is a carboxylic anhydride, preferably cyclic carboxylic anhydride, or a combination of a carboxylic acid with a carbodiimide, preferably EDC.

As the *modified carboxy-terminus cross-linkable modification step* yields a cross-linkable modified carboxy-terminus, preferably polymerizable chemical functional groups are introduced by the modified carboxy-terminus-specific cross-linkable compound to the modified carboxy-terminus.

Thus, the modified carboxy-terminus-specific cross-linkable compound preferably comprises said polymerizable chemical functional groups. Preferably, the polymerizable chemical functional groups remain unaltered in the *modified carboxy-terminus cross-linkable modification step.* Thus, preferably, said polymerizable chemical functional groups do not react with the nucleophile of the modified carboxy-terminus.

Preferably, said polymerizable chemical functional groups contain unsaturated carbon-carbon bonds (-C=C-). Hence, preferably, the modified carboxy-terminus-specific cross-linkable compound comprises cross-linkable moieties selected from the list consisting of a methacrylate group, an itaconyl group, a phloretyl group, a 4-hydroxyphenylacetyl group, and a 3-(4-hydroxyphenyl)propyl group.

In a particularly preferred embodiment, the modified carboxy-terminus-specific cross-linkable compound is an anhydride of a cross-linkable carboxylic acid, preferably methacrylic anhydride, itaconic anhydride, and phloretic anhydride.

In another particularly preferred embodiment, the modified carboxy-terminus-specific cross-linkable compound is a mixture of a carbodiimide, preferably 3-(3-dimethylaminopropyl)carbodiimide, and a cross-linkable carboxylic acid or amide, preferably methacrylic acid, itaconic acid, phloretic acid, 4-hydroxyphenylacetic acid, and 3-(4-hydroxyphenyl)propanoic acid.

In another embodiment, the *modified carboxy-terminus cross-linkable modification step* is carried out using homobifunctional coupling agents as modified carboxy-terminus-specific cross-linkable compounds.

Homobifunctional coupling agents are coupling agents that comprise two individual electrophilic chemical entities of the same type that can react with the nucleophile of the modified carboxy-terminus.

Hence, homobifunctional coupling agents act as crosslinking agents on two peptides comprising a modified carboxy-terminus. Consequently, these two peptides comprising a modified carboxy-terminus are linked to each other via their modified carboxy-termini.

The two chemical entities of the homobifunctional coupling agent are preferably selected from the list consisting of aldehydes, epoxides (oxiranes), isocyanates, isothiocyanates, sulfonyl chlorides, carbodiimides, acyl azides, anhydrides, fluorobenzenes, carbonates, NHS esters, imidoesters, and fluorophenyl esters. These chemical entities are reactive against primary amino groups.

The two chemical entities in the homobifunctional coupling agents can be identical or a combination of items from the aforementioned list.

Most preferably, the homobifunctional coupling agents are selected from the list consisting of 1,4-butanediol diglycidyl ether, glutaraldehyde, hexamethylene diisocyanate, and disuccinimidyl suberate.

The degree of functionalization can be controlled by the amount of modified carboxy-terminus-specific cross-linkable compound used. Preferably, the degree of modification is between 10% to 100%, more preferably between 30% to 100%, even more preferably between 80 % and 100%, and most preferably between 90% and 99%, with respect to the number of carboxy-termini in the peptide.

### e) The N-terminus cross-linkable modification step

In the *N-terminus cross-linkable modification step,* the N-terminus or N-termini of the peptide are chemically functionalized by an N-terminus-specific cross-linkable compound. The N-terminus-specific cross-linkable compound contains electrophilic chemical entities to react with the primary amino groups of the N-terminus or N-termini of the peptide. Thus, the N-terminus-specific cross-linkable compound is a chemical entity, which is highly reactive against primary amines. Hence, the N-terminus-specific cross-linkable compound is selected from the list consisting of aldehydes, epoxides (oxiranes), isocyanates, isothiocyanates, sulfonyl chlorides, carbodiimides, acyl azides, anhydrides, fluorobenzenes, carbonates, NHS esters, imidoesters, and fluorophenyl esters.

In one particularly preferred embodiment, carboxylic acid anhydrides are used as N-terminus-specific cross-linkable compounds. The reaction of the carboxylic acid anhydride and the N-terminus or N-termini having a primary amino group forms an amide bond between the primary amino group and one carboxylic acid moiety from the carboxylic acid anhydride. Thereby, the carboxylic acid anhydride can be selected from cyclic and aliphatic carboxylic acid anhydrides. Cyclic anhydrides originating from a dicarboxylic acid do not generate free carboxylic acid byproducts during this reaction, whereas aliphatic carboxylic acid anhydrides release carboxylic acids as byproducts. Hence, cyclic anhydrides are preferred carboxylic acid anhydrides. However, as the use of anhydrides will always result in the formation of carboxylic groups, which affect the pH value, in the modified carboxy-terminus cross-linkable modification step using anhydrides as modified carboxy-terminus-specific cross-linkable compounds preferably a pH adjustment step is carried out to maintain the deprotonated state of primary amino groups in the peptide.

In another particularly preferred embodiment, a carboxylic acid in combination with a carbodiimide, preferably EDC, is used as N-terminus-specific cross-linkable compounds. This reaction forms an amide bond between the primary amino groups and a carboxylic acid moiety.

Hence, more preferably, in case the nucleophile is a primary amino group, the N-terminus-specific cross-linkable compound is a carboxylic anhydride, preferably cyclic carboxylic anhydride, or a combination of a carboxylic acid with a carbodiimide, preferably EDC.

As the *N-terminus cross-linkable modification step* yields a cross-linkable N-terminus or cross-linkable N-termini, preferably polymerizable chemical functional groups are introduced by the N-terminus-specific cross-linkable compound to the N-terminus or N-termini.

Thus, the N-terminus-specific cross-linkable compound preferably comprises said polymerizable chemical functional groups. Preferably, the polymerizable chemical functional groups remain unaltered in the *N-terminus cross-linkable modification step.* Thus, preferably, said polymerizable chemical functional groups do not react with the nucleophile of the N-terminus.

Preferably, said polymerizable chemical functional groups contain unsaturated carbon-carbon bonds (-C=C-). Hence, preferably, the N-terminus-specific cross-linkable compound comprises cross-linkable moieties selected from the list consisting of a methacrylate group, an itaconyl group, a phloretyl group, a 4-hydroxyphenylacetyl group, and a 3-(4-hydroxyphenyl)propyl group.

In a particularly preferred embodiment, the N-terminus-specific cross-linkable compound is an anhydride of a cross-linkable carboxylic acid, preferably methacrylic anhydride, itaconic anhydride, and phloretic anhydride.

In another particularly preferred embodiment, the N-terminus-specific cross-linkable compound is a mixture of a carbodiimide, preferably 3-(3-dimethylaminopropyl)carbodiimide, and a cross-linkable carboxylic acid or amide, preferably methacrylic acid, itaconic acid, phloretic acid, 4-hydroxyphenylacetic acid, and 3-(4-hydroxyphenyl)propanoic acid.

Most preferably, the N-terminus specific cross-linkable compound and the modified carboxy-terminus-specific cross-linkable compound are identical and the *N-terminus cross-linkable modification step* and the *modified carboxy cross-linkable modification step* are carried out in a combined *termini cross-linkable modification step.*

The degree of functionalization can be controlled by the amount of N-terminus-specific cross-linkable compound used. Preferably, the degree of modification is between 10% to 100%, more preferably between 30% to 100%, even more preferably between 80 % and 100%, and most preferably between 90% and 99%, with respect to the number of N-termini in the peptide.

### f) General remarks

As set out above, most preferably, the method for preparing a telechelic polymer according to the present invention is carried out using a hydrolyzed elastin as peptide.

Since hydrolyzed elastin comprises up to 5%, most preferably less than 3%, amino acids with carboxylic acid side chains, the carboxylic acid side chains in said amino acids also undergo all chemical alterations previously described. This results in peptides containing more than two of said reactive chemical groups that preferably are nucleophiles. Such peptide species are explicitly considered to be an integral component of carboxy-terminally modified elastin peptides.

Preferably, in the method for preparing a telechelic polymer according to the present invention carried out using a hydrolyzed elastin as peptide, the relative number of primary amines in the hydrolyzed elastin comprising a modified carboxy-terminus is increased by a percentage in the range of from 10% to 100%, preferably from 50% to 100%, and most preferably from 75% to 100%, with reference to the hydrolyzed elastin.

In specifically preferred embodiment, the *activated carboxy-terminus nucleophile introduction step* of a hydrolyzed elastin comprising an activated carboxy-terminus peptides is carried out using 4,7,10-trioxa-1,13-tridecanediamine as a compound comprising a nucleophile. In this specifically preferred embodiment, the relative number of primary amines in the hydrolyzed elastin comprising a modified carboxy-terminus is increased by a percentage in the range of from 10% to 200%, preferably form 50% to 200%, and most preferably from 75% to 200%.

### Composition comprising the telechelic polymer

The present invention is further concerned with a composition comprising a telechelic polymer derived from a peptide, wherein the telechelic polymer is suitable for end-crosslinking, and an activatable cross-linking agent.

Generally, the telechelic polymer of the composition according to the present invention is preferably obtainable by the method for preparing a telechelic polymer according to the present invention.

Preferably, the telechelic polymer is derived from a peptide, more preferably derived from a polypeptide. Most preferably, the telechelic polymer is derived from hydrolyzed elastin.

The telechelic polymer is thus a peptide derivative, wherein at least a part of the primary amino groups and/or carboxy groups have been modified by introduction of a cross-linkable group, preferably a cross-linkable group comprising unsaturated carbon-carbon bonds (-C=C-). Most preferably, the telechelic polymer is thus a peptide derivative, wherein at least a part of the primary amino groups and/or carboxy groups have been modified to comprise cross-linkable moieties selected from the list consisting of a methacrylate group, an itaconyl group, a phloretyl group, a 4-hydroxyphenylacetyl group, and a 3-(4-hydroxyphenyl)propyl group. The degree of functionalization of the primary amino groups and/or carboxy groups of the hydrolyzed elastin is preferably between 10% to 100%, more preferably between 30% to 100%, even more preferably between 80 % and 100%, and most preferably between 90% and 99%, with respect to the number of former free primary amino groups and former free carboxylic groups in the peptide.

Moreover, the telechelic polymer is thus a peptide derivative, wherein the former carboxy termini of the peptide comprise a linking unit between the carbonyl group and the cross-linkable group defined above, wherein the linking unit preferably is a carbon chain, optionally comprising hetero atoms. The carbon chain can be saturated or unsaturated. Preferably, the carbon chain is saturated. The hetero atoms are preferably selected from nitrogen and oxygen. Hence, preferably, the carbon chain may comprise ether functional groups and/or amine functional groups, preferably secondary amine functional groups. The length of the carbon chain of the linking unit is preferably in the range of from 3 to 20 carbon atoms and optionally hetero atoms.

The composition may comprise other biocompatible polymers, such as collagen or gelatin. Nevertheless, preferably, the composition comprises only the telechelic polymer as biocompatible polymer, more preferably the only polymer present in the composition is the telechelic polymer.

Preferably, the activatable cross-linking agent comprised in the composition is selected from a photosensitive radical initiator, a thermal radical initiator, or a chemical radical initiator.

The composition may further comprise a solvent. A preferable solvent is water.

### Method for preparing an article

Finally, the present invention concerns a method for preparing an article from a composition according to the present invention, wherein the method is selected from the list consisting of stereolithography, digital light processing, xolography, and continuous liquid interface production. Preferably, stereolithography is selected from masked stereolithography (mSLA); multiphoton lithography, and projection microstereolithography.

### Examples

### a) Reference Example RE1: Preparation of macromolecular elastin

Fresh porcine aorta was obtained from a local slaughterhouse and stored at - 80 °C until use. For the isolation of elastin, the tissue was thawed in 0.9% NaCl and cleared from non-aortic remains. The tissues were chopped and subsequently frozen in liquid nitrogen to facilitate the following pulverization step. 250 mL to 350 mL (i.e. 120 g to 180 g) of pulverized aortic tissue were extracted two times with 1 M NaCl containing 0.02% NaN₃ for 1 h at room temperature under permanent stirring. The washed tissue was subsequently defatted by incubation in 700 mL EtOH and 700 mL acetone for 1 h each. The defatted tissue was dried overnight and hydrolyzed the next day by 0.1 M NaOH for 45 min. The remaining elastin pellet was rinsed with EtOH and dried overnight.

### b) Reference Example RE2: Preparation of elastin hydrolysate via acidic hydrolysation

An elastin hydrolysate containing elastin peptides was prepared by partial acidic hydrolysis of the macromolecular elastin in 0.25 M oxalic acid as described in Example 2 of EP 3 581 212 A1.The elastin hydrolysate was subsequently dialyzed against doubly distilled water for three days using regenerated cellulose (RC) dialysis tubing with a 3.5 kDa molecular weight cut-off (MWCO). The desalted elastin hydrolysate was removed from the dialysis tubing, frozen, and lyophilized to obtain the elastin hydrolysate containing elastin peptides as a dry, off-white to yellowish powder.

### c) Reference Example RE3: Preparation of elastin hydrolysate via alkaline hydrolysation

An elastin hydrolysate containing elastin peptides was prepared by alkaline hydrolysis of macromolecular elastin generating κ-elastin according to Ménasche et al. (1981). The κ-elastin is prepared from porcine aortic insoluble elastin. This elastin is hydrolysed in 1 M KOH in 80% aqueous ethanol (0.5 ml/mg elastin) for 4 h at 37°C. The elastin hydrolysate was subsequently dialyzed against doubly distilled water for three days using regenerated cellulose (RC) dialysis tubing with a 3.5 kDa molecular weight cut-off (MWCO). The κ-elastin solution was subsequently dialyzed against doubly distilled water for three days using RC dialysis tubing with a 3.5 kDa MWCO. The desalted κ-elastin solution was removed from the dialysis tubing, frozen, and lyophilized to obtain the κ-elastin as a dry, white to off-white to faint yellowish powder.

### d) Inventive Example IE1: Preparation of N-(2-Aminoethyl)elastinpeptidylamide (EL-EDA) from elastin hydrolysates generated by acidic hydrolysis

### Batch 1:

3 mL ethylenediamine were dissolved in 10 mL of 0.1 M 2-(N-morpholino)ethanesulfonic acid (MES) buffer and placed in a 100 mL round-bottom flask. The pH was adjusted to 5 using semi-concentrated hydrochloric acid in 0.1 M MES buffer. 2 g of elastin hydrolysate powder prepared according to RE2 were dissolved in 10 mL of MES buffer and added to the reaction flask. The pH was subsequently adjusted to 5.3 and 120 mg of N-(3-dimethylaminopropyl)-N'-ethylcarbodiimide hydrochloride (EDC-HCI) were added. After stirring for one hour at room temperature, an additional 120 mg of EDC-HCI were added, followed by stirring for another two hours. Unreacted educts, reaction side products, and buffer salts were removed by dialysis against distilled water for 72 h using regenerated cellulose (RC) dialysis tubing with a molecular weight cut-off (MWCO) of 3.5 kDa. The solution was then filtered through a folded filter and freeze-dried. The resulting product was obtained as a yellowish powder and comprised of carboxy-terminally modified elastin peptides obtained from an elastin hydrolysate. The relative amount of primary amines was analyzed by ortho-phthalaldehyde (OPA) assay and resulted in an increase of 117%.

### Batch 2:

30 mL ethylenediamine were placed in a 500 mL round-bottom flask. The pH was adjusted to 4.7 using semi-concentrated hydrochloric acid in 0.1 M MES buffer. 20 g of elastin hydrolysate powder prepared according to RE2 were added to the reaction flask and dissolved. The pH was adjusted to 5.2, and 500 mg of EDC-HCI were added. After stirring for one hour at room temperature, the pH was checked and adjusted accordingly to remain in the range of 4 to 6. Another 500 mg of EDC-HCI were then added, and the reaction solution was stirred for additional two hours. Unreacted educts, reaction side products, and buffer salts were removed by dialysis against distilled water for 72 h using a RC dialysis tubing with a MWCO of 3.5 kDa. The solution was then filtered through a folded filter and freeze-dried. The resulting product was obtained as a yellowish powder and comprised of carboxy-terminally modified elastin peptides obtained from an elastin hydrolysate. The relative amount of primary amines was analyzed by OPA assay and resulted in an increase of 80%.

In this example, the carboxy-terminus activation step was implemented by utilizing carbodiimide chemistry and was performed in a "one-pot reaction".

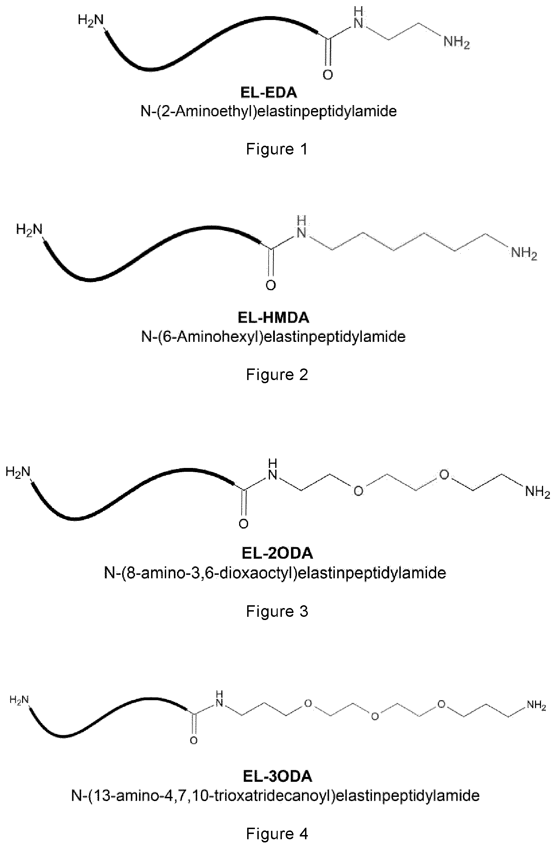

The respective structure of N-(2-Aminoethyl)elastinpeptidylamide (EL-EDA) is depicted in Figure 1. The elastin peptide chain is depictured as a black, bold, and solid line.

### e) Inventive Example IE2: Preparation of N-(2-Aminoethyl)elastinpeptidylamide (κEL-EDA) from elastin hydrolysates generated by alkaline hydrolysis

10 mL ethylenediamine (EDA) and 15 mL of 0.1 M MES buffer were placed in a 100 mL round-bottom flask. The pH was adjusted to 5 using concentrated hydrochloric acid. 5.43 g of κ-elastin powder prepared in RE3 were dissolved in 16 mL of 0.1 M MES buffer and added to the reaction flask. The pH was adjusted to 5.1, and 220 mg of EDC-HCI were added. After stirring for one hour at room temperature, an additional 300 mg of EDC-HCI were added, and the reaction solution was stirred for another two hours. Unreacted educts, reaction side products, and buffer salts were removed by dialysis against distilled water for 48 hours using RC dialysis tubing with a MWCO of 3.5 kDa. The solution was then filtered through a folded filter and freeze-dried. The resulting product was obtained as a yellowish powder and comprised of carboxy-terminally modified elastin peptides obtained from an elastin hydrolysate. The relative amount of primary amines was analyzed by OPA assay and resulted in an increase of 35%.

In this example, the hydrolysis of the elastin was implemented by alkaline hydrolysis. Further, the carboxy-terminus activation step was implemented using carbodiimide chemistry and was carried out in a "one-pot reaction"

The respective structure of κEL-EDA is equivalent to the structure of EL-EDA and thus depicted in Figure 1.

### f) Inventive Example IE3: Preparation of N-(6-Aminohexyl)elastinpeptidylamide (EL-HMDA) from elastin hydrolysates generated by acidic hydrolysis

5.6 g of hexamethylenediamine were dissolved in 10 mL of 0.1 M MES buffer and placed in a 100 mL round-bottom flask. The pH was adjusted to 5 using semi-concentrated hydrochloric acid in 0.1 M MES buffer. 2 g of elastin hydrolysate powder prepared according to RE2 were dissolved in 10 mL of 0.1 M MES buffer and added to the reaction flask. The pH was adjusted to 4.9, and 90 mg of EDC-HCI were added. After stirring for one hour at room temperature, another 90 mg of EDC-HCI were added, followed by stirring for additional two hours. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product was obtained as a yellowish powder and comprised of carboxy-terminally modified elastin peptides obtained from an elastin hydrolysate. The relative amount of primary amines was analyzed by OPA assay and resulted in an increase of 89%.

The respective structure of N-(6-aminohexyl)elastinpeptidylamide (EL-HMDA) is depicted in Figure 2. The elastin peptide chain is depictured as a black, bold, and solid line.

### g) Inventive Example IE4: Preparation of N-(8-amino-3,6-dioxaoctyl)elastinpeptidylamide (EL-2ODA) from elastin hydrolysates generated by acidic hydrolysis

22 mL of 2,2'-(ethylenedioxy)bis(ethylamine) (1,8-diamino-3,6-dioxaoctane) were placed in a 250 mL round-bottom flask. Under ice bath cooling, the pH was adjusted to 6 using concentrated hydrochloric acid. 5.21 g of elastin hydrolysate powder prepared according to RE2 were dissolved in 25 mL of 0.1 M MES buffer and added to the reaction flask. The pH was adjusted to 5.4, and 400 mg of EDC-HCI were added. After stirring for one hour at room temperature, additional 320 mg of EDC-HCI were added, followed by stirring for another two hours. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product was obtained as a yellowish powder and comprised of carboxy-terminally modified elastin peptides obtained from an elastin hydrolysate. The relative amount of primary amines was analyzed by OPA assay and resulted in an increase of 350%.

In this example, 1,8-diamino-3,6-dioxaoctane is reacted with the carboxy-terminally activated peptides.

The respective structure of N-(8-amino-3,6-dioxaoctyl)elastinpeptidylamide (EL-2ODA) is depicted in Figure 3. The elastin peptide chain is depictured as a black, bold, and solid line.

### h) Inventive Example IE5: Preparation of N-(13-amino-4,7,10-trioxatridecanoyl)elastinpeptidylamide (EL-3ODA) from elastin hydrolysates generated by acidic hydrolysis

32 mL of O,O'-bis(3-aminopropyl)diethylene glycol (4,7,10-trioxa-1,13-tridecanediamine) and 25 mL of 0.1 M MES buffer were placed in a 250 mL round-bottom flask. Under ice bath cooling, the pH was adjusted to 6 using concentrated hydrochloric acid. 5 g of elastin hydrolysate powder were dissolved in 25 mL of 0.1 M MES buffer and added to the reaction flask. The pH was adjusted to 5.4, and 320 mg of EDC-HCI were added. After stirring for one hour at room temperature, additional 480 mg of EDC-HCI were added, followed by stirring for another two hours. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product was obtained as a yellowish to light orange powder and comprised of carboxy-terminally modified elastin peptides obtained from an elastin hydrolysate. The relative amount of primary amines was analyzed by OPA assay and resulted in an increase of 155%.

In this example, 4,7,10-trioxa-1,13-tridecanediamine is reacted with the carboxy-terminally activated peptides.

The respective structure of N-(13-amino-4,7,10-trioxatridecanoyl)elastin-peptidylamide (EL-3ODA) is depicted in Figure 4. The elastin peptide chain is depictured as a black, bold, and solid line.

### i) Inventive Example IE6: Preparation of elastin methacrylamide (EL-EDA-MA) from N-(2-Aminoethyl)elastinpeptidylamide (EL-EDA)

Elastin methacrylamide (EL-EDA-MA) was prepared by placing 2 g of EL-EDA as prepared in IE1, batch 2, in a 20 mL vial, adding 15 mL of 0.1 M phosphate buffer (pH 10.7), and shaking until EL-EDA completely dissolved. The pH of the reaction mixture was 9.6. Subsequently, 0.5 mL of methacrylic anhydride was added to the solution and stirred for 2 h at room temperature. Unreacted educts, reaction side products, and buffer salts were removed by dialysis against distilled water for 72 h using RC dialysis tubing with a MWCO of 3.5 kDa. The solution was then freeze-dried. The resulting product, EL-EDA-MA, was obtained as a yellowish to slightly orange powder. The degree of modification (DOM) analyzed by OPA assay was 99%.

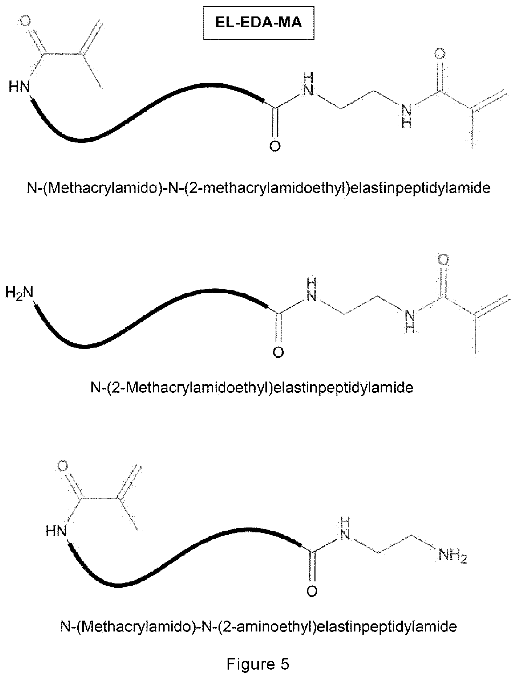

The respective structures of terminally functionalized peptides comprising elastin methacrylamide (EL-EDA-MA) are depicted in Figure 5. The elastin peptide chain is depictured as a black, bold, and solid line. Accordingly, elastin methacrylamide (EL-EDA-MA) prepared from N-(2-Aminoethyl)elastinpeptidylamide (EL-EDA) primarily contains but is not limited to N-(Methacrylamido)-N-(2-methacrylamidoethyl)elastinpeptidylamide, N-(2-Methacrylamidoethyl)-elastinpeptidylamide, and N-(Methacrylamido)-N-(2-aminoethyl)elastinpeptidyl-amide.

### j) Inventive Example IE7: Preparation of elastin methacrylamide (EL-2ODA-MA) from N-(8-Methacrylamido-3,6-dioxaoctyl)elastinpeptidylamide (EL-2ODA)

### Batch 1:

Elastin methacrylamide (EL-2ODA-MA) was prepared by placing 1.92 g of EL-2ODA prepared according to IE4 in a 20 mL vial, adding 15 mL of 0.1 M carbonate buffer (pH 10, filtered), and shaking until EL-2ODA completely dissolved. Subsequently, 0.25 mL of methacrylic anhydride was added to the solution, and stirred for 1 h at room temperature. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product, EL-2ODA-MA, was obtained as a yellowish powder. The degree of modification (DOM) analyzed by OPA assay was 87%.

### Batch 2:

Elastin methacrylamide (EL-2ODA-MA) was prepared by placing 2.08 g of EL-2ODA prepared according to IE4 in a 20 mL vial, adding 15 mL of 0.1 M carbonate buffer (pH 10, filtered), and shaking until EL-2ODA completely dissolved. Subsequently, 0.4 mL of methacrylic anhydride was added to the solution, and stirred for 1 h at room temperature. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product, EL-2ODA-MA, was obtained as a yellowish powder. The degree of modification (DOM) analyzed by OPA assay was 96%.

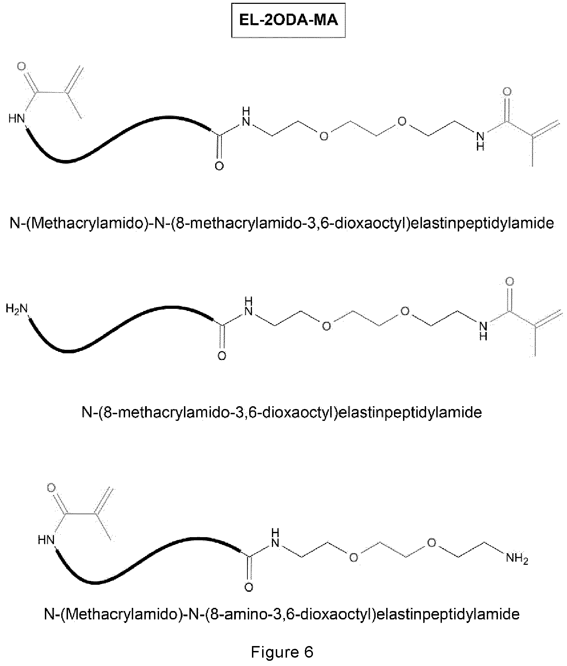

The respective structures of terminally functionalized peptides comprising elastin methacrylamide (EL-2ODA-MA) prepared from EL-2ODA are depicted in Figure 6. The elastin peptide chain is depictured as a black, bold, and solid line. Accordingly, elastin methacrylamide (EL-2ODA-MA) prepared from N-(8-Methacrylamido-3,6-dioxaoctyl)elastinpeptidylamide (EL-2ODA) primarily contains but is not limited to N-(Methacrylamido)-N-(8-methacrylamido-3,6-dioxaoctyl)elastinpeptidylamide, N-(8-methacrylamido-3,6-dioxaoctyl)elastinpep-tidylamide, and N-(Methacrylamido)-N-(8-amino-3,6-dioxaoctyl)elastinpep-tidylamide.

### k) Inventive Example IE8: Preparation of elastin methacrylamide (EL-3ODA-MA) from N-(13-Methacrylamido-4,7,10-trioxatridecyl)elastinpeptidylamide (EL-3ODA)

### Batch 1:

Elastin methacrylamide (EL-3ODA-MA) was prepared by placing 2.08 g of EL-3ODA as prepared in IE5 in a 20 mL vial, adding 15 mL of 0.1 M carbonate buffer (pH 10, filtered), and shaking until EL3ODA completely dissolved. Subsequently, 0.15 mL of methacrylic anhydride was added to the solution, and stirred for 1 h at room temperature. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product, EL-3ODA-MA, was obtained as a yellowish powder. The degree of modification (DOM) analyzed by OPA assay was 69%.

### Batch 2:

Elastin methacrylamide (EL-3ODA-MA) was prepared by placing 2.14 g of EL-3ODA as prepared in IE5 in a 20 mL vial, adding 15 mL of 0.1 M carbonate buffer (pH 10, filtered), and shaking until EL-3ODA completely dissolved. Subsequently, 0.4 mL of methacrylic anhydride was added to the solution, and stirred for 1 h at room temperature. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product, EL-3ODA-MA, was obtained as a yellowish powder. The degree of modification (DOM) analyzed by OPA assay was 90%.

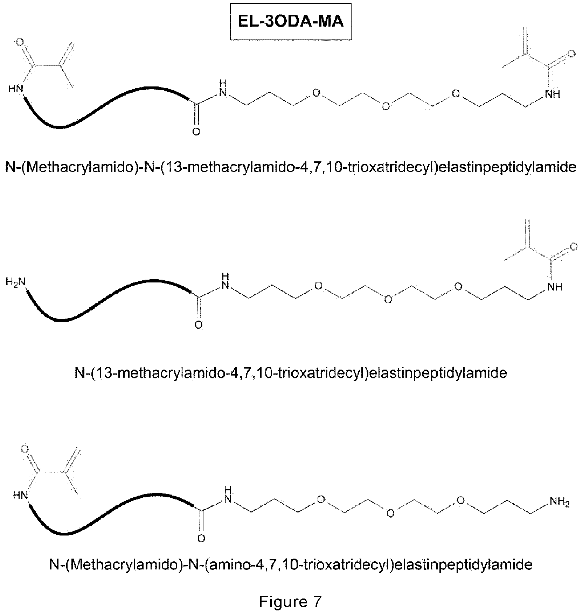

The respective structures of terminally functionalized peptides comprising elastin methacrylamide (EL-3ODA-MA) prepared from EL-3ODA are depicted in Figure 7. The elastin peptide chain is depictured as a black, bold, and solid line. Accordingly, elastin methacrylamide (EL-3ODA-MA) prepared from N-(13-amino-4,7,10-trioxatridecanoyl)elastinpeptidylamide (EL-3ODA) primarily contains but is not limited to N-(Methacrylamido)-N-(13-methacrylamido-4,7,10-trioxatridecyl)-elastinpeptidylamide, N-(Methacrylamido)-N-(13-amino-4,7,10-trioxatridecyl)-elastinpeptidylamide, and N-(13-Methacrylamido-4,7,10-trioxatridecyl)elastin-peptidylamide.

### l) Inventive Example IE9: Preparation of elastin itaconylamide (EL-EDA-IA) from N-(2-Aminoethyl)elastinpeptidylamide (EL-EDA) using itaconic anhydride

Elastin itaconylamide (EL-EDA-IA) was prepared by placing 2 g of EL-EDA prepared according to IE1, batch 2, in a 20 mL vial, adding 15 mL of 0.1 M carbonate buffer (pH 10, filtered), and shaking until EL-EDA completely dissolved. Subsequently, 0.25 g of itaconic anhydride was added to the solution, and stirred for 2 h at room temperature. Unreacted educts, reaction side products, and buffer salts were removed by dialysis against distilled water for 72 h using RC dialysis tubing with a MWCO of 3.5 kDa. The solution was then filtered through a folded filter and freeze-dried. The resulting product, EL-EDA-IA, was obtained as a yellowish powder. The degree of modification (DOM) analyzed by OPA assay was 84%.

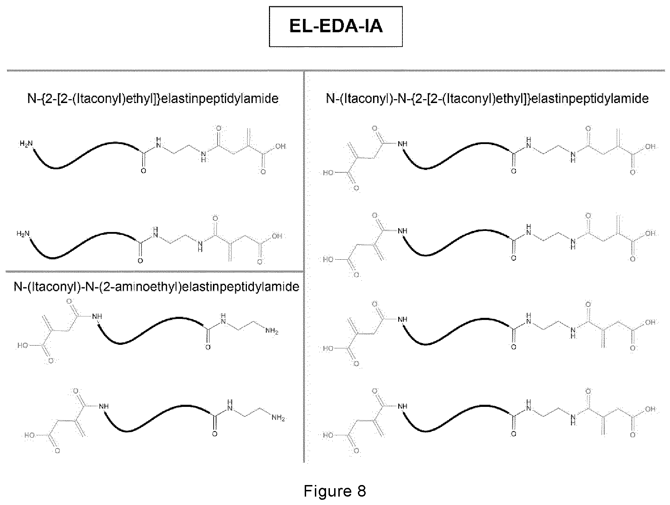

The respective structures of terminally functionalized peptides comprising elastin itaconylamide (EL-EDA-IA) prepared from EL-EDA are depicted in Figure 8. The elastin peptide chain is depictured as a black, bold, and solid line. Accordingly, elastin itaconylamide (EL-EDA-IA) primarily contains but is not limited to N-(Itaconyl)-N-{2-[2-(Itaconyl)ethyl]}elastinpeptidylamide, N-(2-Aminoethyl)-N-(itaconyl)elastinpeptidylamide, and N-(Itaconyl)-N-(2-aminoethyl)elastinpep-tidylamide.

### m) Inventive Example IE10: Photopolymerization of EL-EDA-MA and EL-EDA-IA

EL-EDA-MA and EL-EDA-IA were dissolved in doubly distilled water at concentrations of 10% (w/v) and 20% (w/v), respectively. LAP was added to each solution at a final concentration of 0.1% (w/v). Then, 1 mL of each solution was transferred into a glass vial and placed in a curing station (Prusa CW1). The vials were exposed to 405 nm light until polymerization occurred and the entire liquid phase gelled.

In a separate experiment, a hollow cylindrical structure was generated from a 10% (w/v) EL-EDA-IA solution containing 0.1% (w/v) LAP by reducing the light exposure time in the curing station. The limited penetration depth of the light led to preferential polymerization in the outer regions of the glass vial, resulting in the formation of a hollow cylindrical structure, while the inner regions of the solution remained in a liquid state.

### n) Inventive Example IE11: Thermally induced polymerization of EL-EDA-MA and EL-EDA-IA

EL-EDA-MA and EL-EDA-IA were dissolved at 10% (w/v) in doubly distilled water. Ammonium persulfate was added at a concentration of 0.1% (w/v), and 1 mL of each sample solution was transferred into a glass vial. The samples were incubated in a drying oven at 60 °C until the liquid phase fully gelled.

## Claims

1. A method for preparing a telechelic polymer derived from a peptide, wherein the telechelic polymer is suitable for end-crosslinking, the method comprising the steps of:
- Providing in a *peptide provision step* a peptide, preferably a polypeptide, comprising an N-terminus comprising an amino group and a carboxy-terminus comprising a carboxylic acid group;
- Chemically activating in a *carboxy-terminus activation step* the carboxylic acid group of the carboxy-terminus of said peptide yielding a peptide, preferably a polypeptide, comprising an activated carboxy-terminus;
- Reacting in an *activated carboxy-terminus nucleophile introduction step* said peptide, preferably polypeptide, comprising an activated carboxy-terminus with a compound comprising a nucleophile yielding a peptide, preferably a polypeptide, comprising a modified carboxy-terminus, wherein the modified carboxy-terminus comprises the nucleophile; and
- Reacting in an *N-terminus cross-linkable modification step* said N-terminus with an N-terminus specific cross-linkable compound, thereby yielding a cross-linkable N-terminus;
- Reacting in a *modified carboxy-terminus cross-linkable modification step* said modified carboxy-terminus with a modified carboxy-terminus-specific cross-linkable compound, thereby yielding a cross-linkable modified carboxy-terminus;
wherein the N-terminus cross-linkable modification step can be carried out prior to the modified carboxy-terminus cross-linkable modification step, the N-terminus cross-linkable modification step can be carried out subsequent to the modified carboxy-terminus cross-linkable modification step, or the N-terminus cross-linkable modification step can be carried out at the same time as the modified carboxy-terminus cross-linkable modification step,
thereby yielding a peptide, preferably polypeptide, comprising a cross-linkable N-terminus and a cross-linkable modified carboxy-terminus.

2. The method according to claim 1, wherein the N-terminus specific cross-linkable compound and the modified carboxy-terminus-specific cross-linkable compound are identical and the *N-terminus cross-linkable modification step* and the *modified carboxy cross-linkable modification step* are carried out in a combined *termini cross-linkable modification step.*

3. The method according to claims 1 or 2, wherein the peptide provision step comprises the step of unspecific hydrolysis of a natural occurring polypeptide, preferably unspecific hydrolysis of macromolecular elastin, most preferably unspecific hydrolysis of macromolecular elastin isolated from mammalian tissue, preferably tissue derived from porcine, bovine, human, avian, and fish.

4. The method according to claim 3, wherein the unspecific hydrolysis is selected from the list consisting of acidic hydrolysis, alkaline hydrolysis, enzymatic hydrolysis, or a combination thereof.

5. The method according to any of the preceding claims 1 to 4, wherein the nucleophile is selected from the list consisting of a primary amine, an alcohol, and a thiol.

6. The method according to any of the preceding claims 1 to 5, wherein the compound comprising a nucleophile is a diamine, preferably selected from the list consisting of ethylene diamine, hexamethylene diamine, and 2,2'-(ethylenedioxy)bis(ethylamine).

7. The method according to any of the preceding claims 1 to 6, wherein in the *activated carboxy-terminus nucleophile introduction* the nucleophile is introduced via amide coupling to the activated carboxy-terminus.

8. The method according to any of the preceding claims 1 to 7, wherein the N-terminus specific cross-linkable compound and/or the modified carboxy-terminus-specific cross-linkable compound comprise, preferably consist of, cross-linkable moieties selected from the list consisting of a methacrylate group, an itaconyl group, a phloretyl group, 4-hydroxyphenylacetyl group, and a 3-(4-hydroxyphenyl)propyl group.

9. The method according to any of the preceding claims 1 to 8, wherein the N-terminus specific cross-linkable compound and/or the modified carboxy-terminus-specific cross-linkable compound are selected from aldehydes, epoxides (oxiranes), isocyanates, isothiocyanates, sulfonyl chlorides, carbodiimides, acyl azides, anhydrides, fluorobenzenes, carbonates, NHS esters, imidoesters, and fluorophenyl esters.

10. The method according to claims 8 or 9, wherein the N-terminus specific cross-linkable compound and/or the modified carboxy-terminus-specific cross-linkable compound is an anhydride of a cross-linkable carboxylic acid, preferably methacrylic anhydride, itaconic anhydride, and phloretic anhydride.

11. The method according to claims 8 or 9, wherein the N-terminus specific cross-linkable compound and/or the modified carboxy-terminus-specific cross-linkable compound is mixture of a carbodiimide, preferably 3-(3-dimethylaminopropyl)carbodiimide, and a cross-linkable carboxylic acid or amide, preferably methacrylic acid, itaconic acid, phloretic acid, 4-hydroxyphenylacetic acid, and 3-(4-hydroxyphenyl)propanoic acid.

12. The method according to any of the preceding claims 1 to 11, wherein the *carboxy-terminus activation step* comprises a step selected from the list consisting of formation of an activated ester of the carboxy-terminus, formation of an acyl halides of the carboxy-terminus, activation of the carboxy-terminus by carbodiimides, formation of a mixed anhydride of the carboxy-terminus, and formation of a thioester of the carboxy-terminus, preferably comprises the step of activation of the carboxy-terminus by carbodiimides, activated ester formation, or activated thioester formation.

13. A composition comprising a telechelic polymer derived from a peptide, wherein the telechelic polymer is suitable for end-crosslinking, and an activatable cross-linking agent.

14. The composition according to claim 13, wherein the activatable cross-linking agent is selected from a photosensitive radical initiator, a thermal radical initiator, or a chemical radical initiator.

15. A method for preparing an article from a composition according to claims 13 or 14, wherein the method is selected from the list consisting of stereolithography, digital light processing, xolography, and continuous liquid interface production.
